# EUROPEAN PATENT SPECIFICATION

(11) **EP 1 386 786 B1**
(45) Date of publication and mention of the grant of the patent: **29.06.2005**
(21) Application number: 03015483.5
(22) Date of filing: 09.07.2003
(51) Int. Cl.: B60R 11/04, G08B 21/06

(54) **In-vehicle occupant image pickup apparatus**
Bildaufnahmegerät der Fahrzeuginsassen in einem Fahrzeug
Dispositif d'enregistrement d'images des occupants dans un véhicule

(30) Priority: 26.07.2002 JP 2002218660
(43) Date of publication of application: 04.02.2004
(73) Proprietor: Murakami Corporation, Shizuoka-shi, Shizuoka (JP)
(72) Inventor: Unno, Noriyuki, Murakami Corporation Fujieda, Fujieda-shi, Shizuoka (JP); Sato, Hidenori, Murakami Corporation Fujieda, Fujieda-shi, Shizuoka (JP)
(74) Representative: Strehl Schübel-Hopf & Partner

(56) References cited:
- WO-A-02/08023
- GB-A- 2 371 396
- US-A- 5 278 532
- US-A- 5 291 338
- US-A- 5 786 765
- US-B1- 6 400 835

## Description

### BACKGROUND OF THE INVENTION

This invention relates to in-vehicle occupant image pickup apparatuses, and more particularly to an apparatus capable of picking up an image of an occupant's head in an automobile or other kind of vehicle.

An in-vehicle occupant image pickup apparatus according to the preamble of claim 1 is for instance known from docoment US-B-6 400 835.

In recent years, methoas for detecting inattentive driving and dozing off while driving to raise an alarm has been developed as a technology for supporting safe driving of automobiles. Detection is carried out typically by face orientation, line of sight or eyelid position of a driver, based upon pickup images of the driver's face.

To shoot the face of a driver, Japanese Laid-Open Patent Application, Publication No. 2000-264128 (JP 2000-264128 A) proposes a vehicular interior monitoring device that provides a camera inside the case of an interior rearview mirror of an automobile. In this vehicular interior monitoring device, a mirror body is constructed of a half mirror, through which the camera can shoot the face of the driver.

In the aforementioned vehicular interior monitoring device, the camera is incorporated in the interior rearview mirror which is installed toward the front on one side at an upward and sideward shifted position as viewed from the driver's face, and thus the camera shoots the driver's face in a slanting direction from the upward and sideward shifted position; *i.e.,* the camera cannot shoot a full-faced pose of the driver's face from a direction directly opposite thereto. Accordingly, the device as disclosed in the above document is unfit to precisely detect the face orientation, line of sight and eyelid position of the driver.

In actuality, if a camera could be installed near the instrument panel on the dashboard, the camera could shoot a full-faced pose of the driver's face from a direction directly opposite thereto, but this arrangement would disadvantageously cause the driver some discomfort from the presence of the camera in front of the driver. Moreover, when a camera capable of outputting a digital signal is to be used, installation of such a digital camera near the instrument panel would create another problem of inevitably requiring some change in position of indicating meters because the digital camera to be mounted together with a digital image processor on a substrate needs a large space for installation.

The present invention has been made to eliminate the above-described disadvantages, and it is an exemplified object of the present invention to provide an in-vehicle occupant image pickup apparatus that can shoot the full face of a driver, from a direction directly opposite thereto, without requiring any change in position of the indicating meters in the instrument panel and without causing the occupant any discomfort.

### SUMMARY OF THE INVENTION

In one aspect of the present invention, there is provided an in-vehicle occupant image pickup apparatus which includes an image pickup unit for shooting a face of the driver of the vehicle, and a mirror installed in an instrument panel of a dashboard of the vehicle. The image pickup unit is located in a blind spot for the driver, behind a hood that is provided in the dashboard of the vehicle, but reflection in the mirror enables the image pickup unit to pick up an image of the face of the driver.

With the above in-vehicle occupant image pickup apparatus, the image pickup unit can shoot a full-faced pose of the driver face from a blind spot for the driver, thanks to the mirror installed in the instrument panel which reflects the face. Hereupon, the hood provided in the dashboard is typically a sun visor that is usually provided above the instrument panel, but may be provided as part of the dashboard.

In the in-vehicle occupant image pickup apparatus as described above, preferably, the image pickup unit may include an infrared/near-infrared camera that picks up an image of the face, and an infrared/near-infrared-emitting element that illuminates the face, and the mirror is made up of a cold filter that reflects infrared/near-infrared rays and transmits visible rays. This is because the driver's face can clearly be shot by the infrared/near-infrared camera even in the nighttime, with the help of illumination of the infrared/near-infrared rays reflected off the cold filter. Alternatively, the mirror may be made up of a half mirror.

The above image pickup unit may include a camera that picks up an image of the face and outputs a digital image signal, and a digital image processor that manipulates the digital image signal, whereas the camera and the digital image processor are both mounted on one and the same substrate. Such an arrangement allows an image of the driver's face captured in the camera to be provided in a noiseless condition to undergo digital processing in the digital image processor.

The above "infrared/near-infrared" is intended to mean at least one of "infrared" and "near-infrared"; that is, three cases conceivable is: where to include infrared only, where to include near-infrared only, and where to include both of infrared and near-infrared.

Other objects and further features of the present invention will become readily apparent from the following description of preferred embodiments with reference to accompanying drawings.

### BRIEF DESCRIPTION OF THE DRAWINGS

FIG. 1 is a schematic diagram, in cross section, showing an arrangement of one exemplary embodiment of an in-vehicle occupant image pickup apparatus according to the present invention.
FIG. 2 is a schematic plan view showing an arrangement of an image pickup unit as shown in FIG. 1.
FIG. 3 is a cross section showing a structure of a cold filter as shown in FIG. 1.
FIG. 4 is a graph showing transmittance of the cold filter as shown in FIG. 1.

### DETAILED DESCRIPTION OF THE PREFERRED EMBODIMENTS

A description will be given of exemplified embodiments of the in-vehicle occupant image pickup apparatus according to the present invention with reference to the drawings.

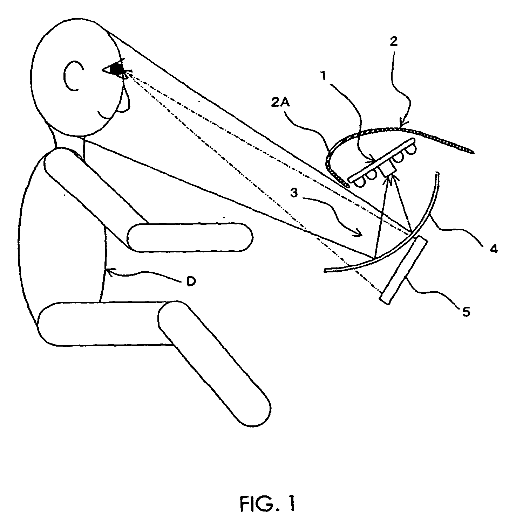

The in-vehicle occupant image pickup apparatus according to the present invention is exemplified by an embodiment, as shown in FIG. 1, which includes an image pickup unit 1 capable of shooting the face of a driver D as an example of an occupant of an automobile or other kind of vehicle. The image pickup unit 1 is located in a blind spot for the occupant (driver D) behind a hood 2A of a dashboard 2, and a cold filter 4 serving as a mirror is provided in a instrument panel 3 to enable the image pickup unit 1 to pick up an image of the face of the driver D reflected therefrom.

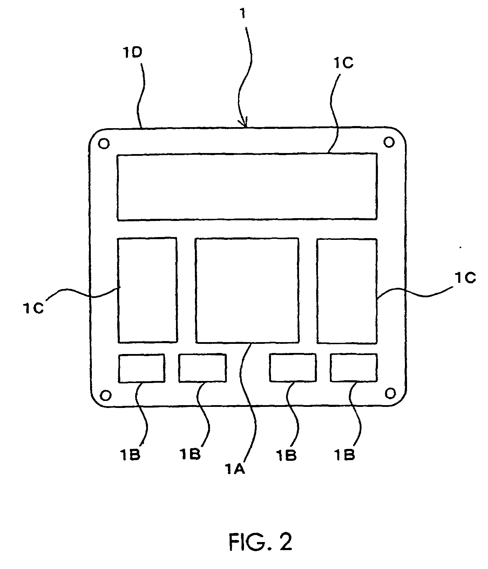

As shown in FIG. 2, the image pickup unit 1 includes an infrared/near-infrared camera 1A that picks up an image and outputs a digital image signal, a plurality of infrared/near-infrared-emitting diodes 1B (equivalent to the above infrared/near-infrared-emitting element) that illuminate the face, and an image signal processor 1C that manipulates the digital image signal outputted from the infrared/near-infrared camera 1A, all of which are mounted on one and the same substrate 1D. The image pickup unit 1 is tilted so that the infrared/near-infrared camera 1A for outputting a digital image signal and the infrared/near-infrared-emitting diodes 1B may face the instrument panel 3 and may be opposed to the cold filter 4.

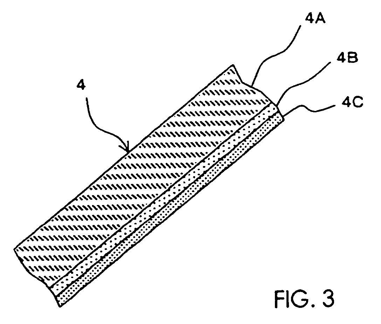

The above cold filter 4 is put over indicating meters 5 provided in the instrument panel 3. The cold filter 4 is, as shown in FIG. 3, made up of a glass substrate 4A of which a backside is coated with a thin TiO₂ layer 4B and a thin SiO₂ layer 4C.

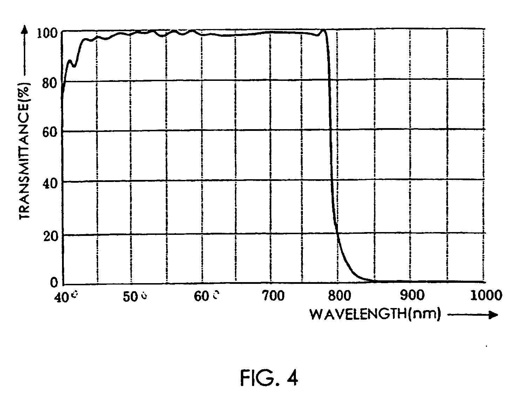

The cold filter 4 has light-transmittance characteristics as shown in FIG. 4. Specifically, infrared rays (including near-infrared rays) with wavelengths equal to or greater than 800nm are generally not transmitted but reflected, while nearly 100% of visible rays with wavelengths less than 800nm are transmitted. In addition, the cold filter 4 is, as shown in FIG: 1, curved in cross section with a specific curvature that permits the infrared/near-infrared rays emitted from the infrared/near-infrared-emitting diodes 1B of the image pickup unit 1 to be reflected toward the face of the driver D, and the infrared/near-infrared rays from the face of the driver D to be reflected toward the infrared/near-infrared camera 1A of the image pickup unit 1 where the captured images are outputted in the form of a digital image signal.

According to the above-described embodiment of the in-vehicle image pickup apparatus, the digitized image signal-outputting infrared/near-infrared camera 1A of the image pickup unit 1 located in a blind spot for the driver D behind the hood 2A of the dashboard 2 picks up images of a full-faced pose of the face of the driver D while the driver D drives the automobile, with the help of the infrared/near-infrared rays reflected off the cold filter 4. During night driving, illumination of infrared/near-infrared rays emitted from the infrared/near-infrared-emitting diodes 1 B of the image pickup unit 1 and reflected off the cold filter allows the digitized image signal-outputting infrared/near-infrared camera 1A to clearly pick up images of the face of the driver D.

Digital image signals of images picked up by the digitized image signal-outputting infrared/near-infrared camera 1A are manipulated by the image signal processor 1C of the image pickup unit 1, and outputted to, for example, a safe driving support control device (not shown) of the automobile. The safe driving support control device accurately detects the face orientation, line of sight and eyelid position of the driver D, so that the safe driving support control device may raise an alarm to call attention of the driver D when detecting that the driver D is looking off the road or dozing off while driving.

In this embodiment of the in-vehicle occupant image pickup apparatus, the cold filter 4 transmits visible rays, and thus the driver D or fellow passengers can see the indicating meters 5 inside the instrument panel 3 through the cold filter 4. Consequently, the indicating meters 5 need not be changed in position, so that the in-vehicle occupant image pickup apparatus can be arranged and installed at low cost.

Moreover, since the image pickup unit 1 including the digitized image signal-outputting infrared/near-infrared camera 1A is installed in a blind spot for the driver D and other occupants behind the hood 2A of the dashboard 2, the driver D and other occupants would not feel any discomfort. Further, since the images of the driver's face can be shot from a position near the driver D and from a direction directly opposite thereto, the face of the driver D or other occupants can stably be shot in a natural state from the direction directly opposite thereto.

Furthermore, since the image pickup unit 1 has the digitized image signal-outputting infrared/near-infrared camera 1A, the infrared/near-infrared-emitting diodes 1B and the image signal processor 1C all mounted on the substrate 1D, the images of the face of the driver D captured by the digitized image signal-outputting infrared/near-infrared camera 1A can be provided as is in a noiseless condition for a subsequent digital image processing, so that the face orientation, line of sight and eyelid position of the driver D can accurately be detected at low cost. As the digitized image signal-outputting infrared/near-infrared camera 1A, the infrared/near-infrared-emitting diodes 1B and the image signal processor 1C are mounted on one and the same substrate 1D, the image pickup unit 1 can be provided at low cost.

Although the one preferred embodiment of the present invention have been described above, the present invention is not limited to the above embodiment, and various modifications and changes may be made in the in-vehicle occupant image pickup apparatus according to the present invention without departing from the spirit and scope thereof. For example, the cold filter 4 as a mirror, which is curved in the above embodiment, may be provided in the form of a flat plate.

The cold filter 4 may be substituted with a half mirror. In this instance, the digitized image signal-outputting infrared/near-infrared camera 1A is replaced with a camera that picks up images with visual rays and outputs a digital signal or an analog signal.

As described above, the in-vehicle occupant image pickup apparatus according to the present invention is designed to allow an image pickup unit located in a blind spot for an occupant behind a hood of the dashboard to shoot a full face of the occupant from a direction directly opposite thereto with the help of reflection in a mirror installed in an instrument panel. According to the present invention, thus, the full face of the occupant can be shot from a direction directly opposite thereto without requiring any change in position of the indicating meters in the instrument panel and without causing the occupant any discomfort, and the face orientation, line of sight and eyelid position of the occupant can be precisely detected.

As illustrated in one aspect of the present invention, if the image pickup unit includes an infrared/near-infrared camera that picks up an image of the face, and an infrared/near-infrared-emitting element that illuminates the face, and the mirror is made up of a cold filter that reflects infrared/near-infrared rays and transmits visible rays, the occupant's face can clearly be shot by the infrared/near-infrared camera even in the nighttime, with the help of illumination of the infrared/near-infrared rays reflected off the cold filter, while the occupant can see the indicating meters through the cold filter.

If a digital image processor is mounted on the substrate on which the camera that picks up an image of the face and outputs a digital image signal, the image of the occupant's face picked up by the camera can be provided as is in a noiseless condition for a subsequent digital image processing, so that the face orientation, line of sight and eyelid position of the driver can accurately be detected at low cost.

## Claims

1. An in-vehicle occupant image pickup apparatus comprising:
an image pickup unit (1) for shooting a face of the driver (D) of the vehicle, **characterized in that** the image pickup unit (1) is located in a blind spot for the driver (D), behind a hood (2A) that is provided in the dashboard (2) of the vehicle; and **in that** the image pickup apparatus further comprises a mirror (4) installed in an instrument panel (3) of the dashboard (2), reflection in the mirror (4) enabling the image pickup unit (1) to pick up an image of the face of the driver (D).

2. An in-vehicle occupant image pickup apparatus according to claim 1, wherein the image pickup unit (1) includes an infrared/near-infrared camera (1A) that picks up an image of the face of the driver (D), and an infrared/near-infrared-emitting element (1B) that illuminates the face of the driver (D); and wherein the mirror is made up of a cold filter (4) that reflects infrared/near-infrared rays and transmits visible rays.

3. An in-vehicle occupant image pickup apparatus according to claim 1, wherein the image pickup unit (1) includes a camera (1A) that picks up an image of the face of at driver (D); and
wherein the mirror is made up of a half mirror (4).

4. An in-vehicle occupant image pickup apparatus according to any one of claims 1 through 3, wherein the image pickup unit (1) includes:
a camera (1A) that picks up an image of the face of the driver (D) and outputs a digital image signal;
a digital image processor (1C) that manipulates the digital image signal; and
a substrate (1D) on which the camera (1A) and the digital image processor (1C) are both mounted.

5. An in-vehicle instrument panel unit which is equipped with an in-vehicle image pickup apparatus according to any one of claims 1 through 3.

## Patentansprüche

1. Vorrichtung zum Aufnehmen eines Bildes eines Fahrzeuginsassen, umfassend:
eine Bildaufnahmeeinheit (1) zum Fotografieren des Gesichts des Fahrers (D) des Fahrzeugs, **gekennzeichnet dadurch, daß** die Bildaufnahmeeinheit (1) an einer für den Fahrer (D) nicht einsehbaren Stelle hinter einer im Armaturenbrett (2) des Fahrzeugs vorgesehenen Blende (2A) angeordnet ist; und **dadurch**, daß die Bildaufnahmevorrichtung ferner umfaßt:
einen in einer Instrumententafel (3) des Armaturenbretts (2) installierten Spiegel (4), wobei die Reflexion des Spiegels (4) der Bildaufnahmeeinheit (1) die Aufnahme eines Bildes des Gesichts des Fahrers (D) ermöglicht.

2. Vorrichtung zum Aufnehmen eines Bildes eines Fahrzeuginsassen nach Anspruch 1, wobei die Bildaufnahmeeinheit (1) eine Infrarot/Nah-Infrarot-Kamera (1A) enthält, die ein Bild des Gesichts des Fahrers (D) aufnimmt, sowie ein Infrarot/Nah-Infrarot-ausstrahlendes Element (1B), daß das Gesicht des Fahrers (D) erleuchtet; und
wobei der Spiegel aus einem kalten Filter (4) hergestellt ist, das Infrarot/Nah-Infrarot-Strahlen reflektiert und sichtbare Strahlen überträgt.

3. Vorrichtung zum Aufnehmen eines Bildes eines Fahrzeuginsassen nach Anspruch 1, wobei die Bildaufnahmeeinheit (1) eine Kamera (1A) umfaßt, die ein Bild des Gesichts des Fahrers (D) aufnimmt; und
wobei der Spiegel aus einem Halbspiegel (4) hergestellt ist.

4. Vorrichtung zum Aufnehmen eines Bildes eines Fahrzeuginsassen nach einem der Ansprüche 1 bis 3, wobei die Bildaufnahmeeinheit umfaßt:
eine Kamera, die ein Bild des Gesichts aufnimmt und ein digitales Bildsignal ausgibt;
einen Digitalbildprozessor, der das digitale Bildsignal manipuliert; und
ein Substrat, auf dem sowohl die Kamera als auch der Digitalbildprozessor befestigt sind.

5. Eine Instrumententafeleinheit eines Fahrzeugs, die mit einer Vorrichtung zur Aufnahme eines Bildes eines Fahrzeuginsassen nach einem der Ansprüche 1 bis 3 ausgestattet ist.

## Revendications

1. Appareil de prise de vue d'image d'occupant dans un véhicule comprenant :
une unité de prise de vue d'image (1) pour effectuer une prise de vue du visage d'un conducteur (D) du véhicule, **caractérisé en ce que** l'unité de prise de vue d'image (1) est située dans un endroit aveugle pour le conducteur (D) derrière un capuchon (2A) qui est placé dans le tableau de bord (2) du véhicule ; et **en ce que** l'appareil de prise de vue d'image comprend en outre un miroir (4) installé dans un tableau d'instrumentation (3) du tableau de bord (2), la réflexion dans le miroir (4) permettant à l'unité de prise de vue d'image (1) d'effectuer une prise de vue d'une image du visage du conducteur (D).

2. Appareil de prise de vue d'image d'occupant dans un véhicule selon la revendication 1, dans lequel l'unité de prise de vue d'image (1) comprend une caméra infrarouge/proche infrarouge (1A) qui effectue une prise de vue d'une image du visage du conducteur (D) dans un élément d'émission infrarouge/proche infrarouge (1B) qui illumine le visage du conducteur (D) ; et
dans lequel le miroir est constitué d'un filtre froid (4) qui reflète des rayons du domaine de l'infrarouge/proche infrarouge et émet des rayons visibles.

3. Appareil de prise de vue d'image d'occupant dans un véhicule selon la revendication 1, dans lequel l'unité de prise de vue d'image (1) comprend une caméra (1A) qui effectue une prise de vue d'une image du visage du conducteur (D) ; et
dans lequel le miroir est constitué d'un semi-miroir (4).

4. Appareil de prise de vue d'image d'occupant dans un véhicule selon l'une quelconque des revendications 1 à 3, dans lequel l'unité de prise de vue d'image (1) comprend :
une caméra (1A) qui effectue une prise de vue d'une image du visage du conducteur (D) et fournit un signal d'image numérique ;
un processeur de signal d'image numérique (1C) qui manipule le signal d'image numérique ; et
un substrat (1D) sur lequel la caméra (1A) et le processeur de signal d'image numérique (1C) sont les deux montés.

5. Unité d'un tableau d'instrumentation dans un véhicule qui est équipé d'un appareil de prise de vue d'image de véhicule selon une quelconque des revendications 1 à 3.
